# EUROPEAN PATENT SPECIFICATION

(11) **EP 1 818 052 B1**
(45) Date of publication and mention of the grant of the patent: **11.11.2009**
(21) Application number: 05763090.7
(22) Date of filing: 28.06.2005
(51) Int. Cl.: A61K 31/167, A61P 35/00, A61P 31/18, A61P 31/00, A61P 31/10, A61P 11/08, A61P 9/00, A61P 19/02

(54) **USE OF FORMOTEROL IN THE PROPHYLACTIC AND/OR THERAPEUTIC TREATMENT OF MUSCLE WASTING AND/OR CACHECTIC SYNDROME WHICH ARE ASSOCIATED WITH CATABOLIC CONDITIONS OF CERTAIN DISEASES, SUCH AS CANCER, AIDS, INFECTIONS, DIABETES AND OTHERS**
VERWENDUNG VON FORMOTEROL IN DER PROPHYLAKTISCHEN UND/ODER THERAPEUTISCHEN BEHANDLUNG VON MUSKELSCHWUND UND/ODER KACHEXIE-SYNDROM IM ZUSAMMENHANG MIT KATABOLISCHEN ZUSTÄNDEN BEI BESTIMMTEN KRANKHEITEN, WIE Z.B. KREBS, AIDS, INFEKTIONEN, DIABETES UND ANDERE
UTILISATION DU FORMOTEROL DANS LE TRAITEMENT PROPHYLACTIQUE ET/OU THERAPEUTIQUE DE L'USURE MUSCULAIRE ET/OU DU SYNDROME CACHECTIQUE, ASSOCIES A DES ETATS CATABOLIQUES DE CERTAINES MALADIES TELLES QUE LE CANCER, LE SIDA, LES INFECTIONS, LE DIABETE ET AUTRES MALADIES DE CE TYPE

(30) Priority: 29.06.2004 ES 200401941
(43) Date of publication of application: 15.08.2007
(73) Proprietor: Acacia Pharma Limited, Harston Cambridgeshire CB22 7GG (GB)
(72) Inventor: ARGILÉS HUGUET, José María, E-08190 Valldoreix (ES); LÓPEZ SORIANO, Francisco Javier, E-08028 Barcelona (ES); BUSQUETS RIUS, Sílvia, E-08789 Vilanova d'Espoia (ES)
(74) Representative: Perry, Robert Edward
(86) International application number: PCT/ES2005/000365
(87) International publication number: WO 2006/003222

(56) References cited:
- WO-A1-01/32163
- WO-A1-02/066027
- ES-A1- 2 178 943
- BUSQUETS S ET AL: 'Anticachectic effects of formoterol: a grug for potential treatment of muscle wasting' CANCER RESEARCH vol. 64, no. 18, 15 September 2004, pages 6725 - 6731, XP003013527

## Description

This invention relates to the field of human medicine, and specifically to a new use of a compound for the treatment of particular human diseases or states.

### BACKGROUND ART

Perhaps the most common manifestation of advanced malignant disease is the development of cancer cachexia. Indeed, cachexia occurs in the majority of cancer patients, and it is responsible for the death of 22% of cancer patients. In addition to cancer, cachexia occurs associated with other diseases or states such as acquired immune deficiency syndrome (AIDS), traumatism or sepsis. The abnormalities associated with cachexia include anorexia, weight loss, muscle loss and atrophy, anemia and alterations in carbohydrate, lipid and protein metabolism (cf. Argilés J.M. et al., "The metabolic basis of cancer cachexia", Med. Res. Rev. 1997, vol. 17, pp. 477-98). The degree of cachexia is inversely correlated with the survival time of the patient and it always implies a poor prognosis. One of the most relevant characteristics of cachexia is asthenia (i.e. lack of muscular strength), which reflects the important muscle waste that takes place in the cachectic cancer patient. Asthenia is also characterized by a general weakness as well as physical and mental fatigue. In addition, lean body mass depletion is one of the main trends of cachexia and it involves not only skeletal muscle but it also affects cardiac proteins, resulting in important alterations in heart performance.

Several strategies have been proposed to treat cachexia, although only few of them have arrived to a clinical level with positive results. However, most of these have shown troubles derived of their utilization. Among the most widely used anticachectic drugs are several synthetic progesterone derivatives (e.g. megestrol acetate and medroxyprogesterone), which are able to improve appetite and weight gain, although the observed increase in body mass is mainly due to an increased fat mass and water retention, without significant effects on the Karnofsky index (a measure of activity level for cancer patients). The cannabinoid dronabinol is able to improve cachexia both in cancer and AIDS patients, although many of them experience side effects such as euphoria, dizziness, somnolence and confusion. Insulin has been used as an appetite enhancer (in order to improve anorexia), but it has been shown to be associated to an increase in tumor weight. Corticosteroids (e.g. dexametasone or prednisolone), when used in prolonged treatments, seem to lead weakness, delirium, osteoporosis and immunosuppression, and they do not seem to have any significant effects on the reduction of mortality. Clenbuterol is another compound that has shown some anti-cachectic effect, but its toxicity in humans and animals (specially its negative effects on heart) has not allowed the undertaking of clinical trials (cf. Carbó N. et al.,"Comparative effects of beta2-adrenergic agonists on muscle waste associated with tumor growth", Cancer Lett. 1997, vol. 115, pp. 113-8; Hoffman, R.J. et al., "Clenbuterol ingestion causing prolonged tachycardia, hypokalemia, and hypophosphatemia with confirmation by quantitative levels", J. Toxicol. Clin. Toxicol. 2001, vol. 39, pp. 339-44). The use of antibodies and soluble receptors of cytokines is a promising approach to treat cachexia, but these treatments are too expensive because they require a very large number of molecules in order to block cytokine action completely. The same happens with the use of anti-inflammatory cytokines. The use of anabolic steroids (e.g. nandrolone and oxymetholone) has important adverse side effects such as masculinization, fluid retention and hepatic toxicity. Hydrazine sulfate has been used as an anticachectic drug, but it has some problems derived from its toxicity (cf. Argilés J.M. et al.,"Cancer cachexia: a therapeutic approach", Med. Res. Rev. 2001, vol. 21, pp. 83-101).

Thus, it is highly desirable to provide new therapeutic agents for the treatment of cachexia and muscle wasting. In particular, it is desirable to have new therapeutic agents with a great anti-cachectic efficiency and non-toxic consequences.

### SUMMARY OF THE INVENTION

Inventors have surprisingly found that formoterol, used until now for the treatment of bronchospasm associated with asthma, exhibits a powerful anti-cachectic efficiency with relatively low adverse side effects and low toxicity.

Formoterol is a racemic compound which has the accompanying chemical formula (showing the relative stereochemistry of one of the two enantiomers) and it is a highly potent β₂-adrenoceptor-selective agonist which combines the clinical advantages of a rapid onset of action and a long duration of action. This compound is used in humans for the treatment of bronchospasm associated with asthma. In vitro formoterol is a potent airway smooth muscle relaxant with high efficacy, and very high affinity and selectivity for the β₂-adrenoceptor. In addition to airway smooth muscle relaxation, formoterol inhibits a range of acute inflammatory processes in animal models, including eosinophil activation and infiltration of the airways, microvascular leakage, and antigen-induced mediator release from human lung. Moreover, formoterol relaxes bronchial smooth muscle and also provides important clinical benefits in symptomatic patients with chronic obstructive pulmonary disease (COPD). Formoterol, as other long-acting β₂-adrenoceptor agonists, attenuates the allergen-induced late asthmatic reaction and, under certain conditions, it has more effect than other β₂-adrenoceptor agonists such as salbutamol and salmeterol (cf. Linden A. et al., "Salmeterol, formoterol and salbutamol in the isolated guinea pig trachea: differences in maximum relaxant effect and potency but not in functional antagonism" Thorax 1993, vol. 48, pp. 547-53).

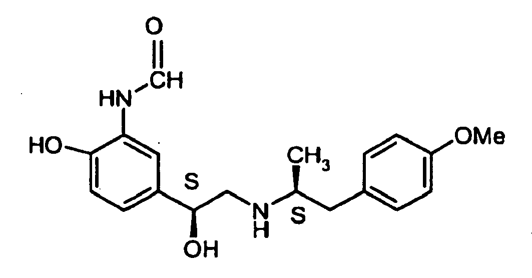

The present invention relates to the use of formoterol or a pharmaceutically acceptable salt thereof, or a pharmaceutically acceptable solvate of any of them, for the preparation of a medicament for the prophylactic and/or therapeutic treatment of muscle wasting and/or a cachectic syndrome, being both associated to a catabolic state in a mammal, in particular a human. The term "catabolic state" as used herein, means a pathophysiological situation in which the catabolic pathways are activated. Examples of catabolic pathways are hepatic glycogenolysis, adipose tissue lipolysis, skeletal muscle proteolysis and thermogenesis. Thus, the invention can be used for treatment of a patient suffering from muscle wasting and/or a cachectic syndrome both of them associated to a catabolic state in a mammal, in particular a human, comprising the administration of a therapeutical acceptable dose of formoterol or their pharmaceutically acceptable salts and/or solvates, together with pharmaceutically acceptable excipients. In a particular embodiment, formoterol fumarate, as such or as dihydrate, is used for these therapeutical indications.

Formoterol exerts a selective, powerful, protective action on heart and skeletal muscle by antagonizing the enhanced protein degradation that characterizes cancer cachexia. Thus, conversely to what it is found with other β₂-agonists that have numerous side effects and considerable toxicity in humans, formoterol is a convenient therapeutic agent in pathological states wherein muscle protein hypercatabolism is a critical feature, such as cancer cachexia or other muscle wasting diseases.

In particular embodiments of the invention, formoterol or their pharmaceutically acceptable salts and/or solvates can be used for the treatment of muscle wasting or cachectic syndrome associated to cancer, acquired immune deficiency syndrome (AIDS), sepsis, diabetes, immobilization states, severe burns, chronic obstructive pulmonary disease, aging, cardiovascular pathologies, hepatic pathologies, rheumatoid arthritis, nutrition disorders, acidosis, neuromuscular diseases, Alzheimer disease, hyperadrenocortisolism, hyperthyroidism, Cushing's syndrome, muscle atrophies and distrophies, ulcerative colitis, chronic fatigue syndrome, renal failure, liver cirrhosis, Crohn's disease and a muscle wasting state due to weightlessness.

Treatment with formoterol has a clear protective effect for skeletal muscle and for the heart, in terms of wet tissue weight. Then, formoterol provides a significant trophic action on muscles in non-tumor bearers as well, particularly on the gastrocnemius, tibialis, EDL (extensor digitorum longus) and cardiac muscle, while the soleus was not significantly affected. Regarding adipose tissues, formoterol treatment induces an increase in brown adipose tissue weight. Furthermore, formoterol treatment do not alter either the weight of the solid tumor or the food intake.

Depending on circumstances, a person skilled in the art would select an appropriate administration via of formoterol or their pharmaceutically acceptable salts and/or solvates, such as oral, nasal, parenteral, subcutaneous, topical, etc. The person skilled in the art would also choose a suitable delivery system for the selected administration.

The following particular embodiments and drawings are provided by way of illustration, and they are not intended to be limiting of the present invention.

### DESCRIPTION OF THE DRAWINGS

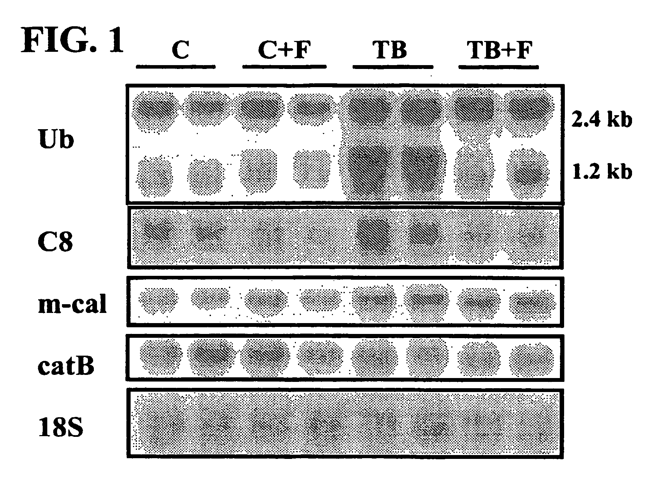

FIG. 1 shows the Northern blot of gastrocnemius muscles from control rats (C), formoterol-treated rats (C+F), tumor-bearing rats (Yoshida AH-130 ascites hepatoma) (TB) and formoterol-treated tumor-bearing rats (TB+F). Expression of ubiquitin (Ub), C8 proteasome subunit (C8), m-calpain (m-cal) and cathepsin B (catB) mRNA in gastrocnemius was detected after hybridization with cDNA probes. A 18S rRNA probe (18S) was used for total quantification and loading correction. Autoradiographs were subjected to scanning densitometry.

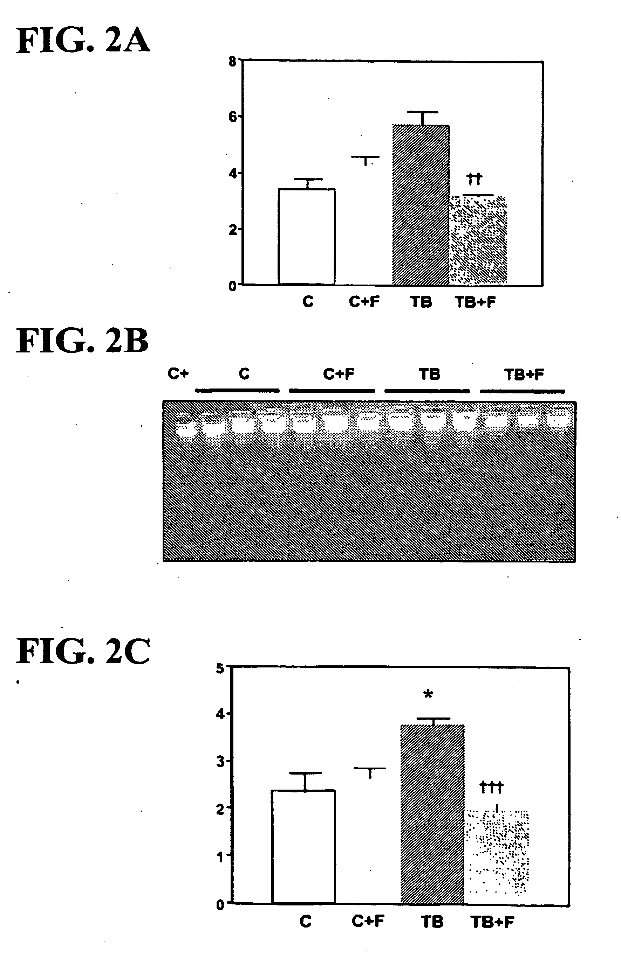

FIG. 2 shows apoptosis in tibialis muscles from control rats (C), formoterol-treated rats (C+F), tumor-bearing rats (Yoshida AH-130 ascites hepatoma) (TB) and formoterol-treated tumor-bearing rats (TB+F). Results are mean ± SEM for five animals in each group. FIG. 2A and 2B: DNA fragmentation was assessed by monitoring the laddering in an agarose gel. The percentage of DNA fragmentation was quantified by scanning densitometry. Liver from anti-Fas antibody-treated mice (C+) was used as positive control. FIG. 2C: caspase-3 activity was determined by a colorimetric assay, and the results are expressed as nmol/hour. Statistical significance of the results (by Student's t test); FIG. 2A: untreated versus F: ttp<0.01; FIG. 2C: C versus TB: *p<0.05, untreated versus F: †††p<0.001.

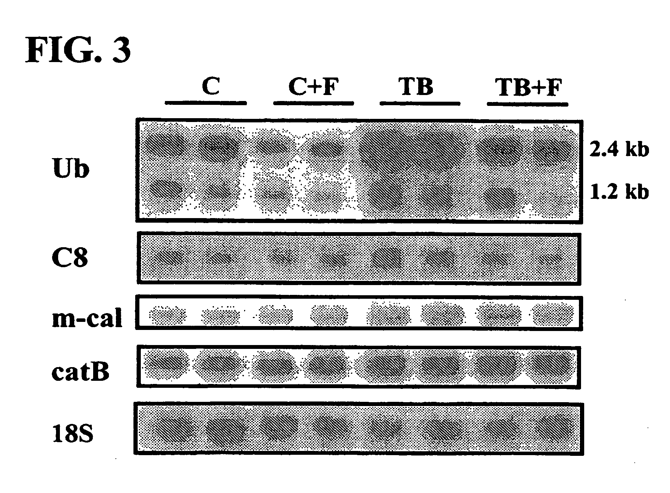

FIG. 3 shows the Northern blot of gastrocnemius muscles from control mice (C), formoterol-treated mice (C+F), tumor-bearing mice (Lewis lung carcinoma) (TB) and formoterol-treated tumor-bearing mice (TB+F). The procedure followed and abbreviations are analogous to those in FIG. 1.

### DETAILED DESCRIPTION OF PARTICULAR EMBODIMENTS

### Animals and biochemicals

Male Wistar rats (Interfauna, Barcelona, Spain), weighting about 110 g, and C57BI/6 mice (Criffa, Barcelona, Spain), of about 12 weeks of age, were used in the different experiments. The animals were maintained at 22 ± 2 °C with a regular light-dark cycle (light on from 08:00 a.m. to 08:00 p.m.) and had free access to food and water. The diet (B.K. Universal G.J./S.L., Sant Vicenç del Horts, Barcelona, Spain) consisted of 45.5-48.5% carbohydrate (3.5% absorbable glucose, 42-45% starch), 18.5% protein and 3.1% fat (the residue was non digestible material). The food intake was measured daily. All animal manipulations were made in accordance with the European Community guidelines for the use of laboratory animals. Biochemicals were all reagent grade and obtained either from Roche S.A. (Barcelona, Spain) or from Sigma Chemical Co. (St. Louis, MO, USA). Radiochemicals were purchased from Amersham Int. (Amersham, Bucks., UK) and formoterol fumarate micronized by Industriale Chimica s.r.l. (Saronno, Italy).

### Tumor inoculation and treatment

Yoshida AH-130 ascites hepatoma: This is a rat tumor that grows exponentially for 4-5 days and shifts into a stationary phase by day 7 after transplantation. Rats were divided into two groups, namely controls and tumor hosts. The latter received an intraperitoneal inoculum of 10⁸ AH-130 Yoshida ascites hepatoma cells obtained from exponential tumors. Both groups were further divided into treated and untreated, the former being administered a daily intraperitoneally (i.p.) dose of formoterol (2 mg/kg body weight (bw), dissolved in physiological solution) and the latter a corresponding volume of solvent. On day 7 after tumor transplantation, the animals were weighted and anesthetized with an i.p. injection of ketamine/xylazine mixture (3:1) (Imalgene^{®} and Rompun^{®} respectively). The tumor was harvested from the peritoneal cavity and its volume and cellularity evaluated. Blood was collected from the abdominal aorta into heparinized tubes and centrifuged (3500 g, 10 min, 4 °C) to obtain plasma. Tissues were rapidly excised, weighted, and frozen in liquid nitrogen.

In the combination treatment of formoterol and pentoxifylline, the experimental groups were the following: control rats and tumor-bearing rats, two, four and seven days after tumor inoculation. Both groups were further divided into treated and untreated, the former being administered with a daily subcutaneously (s.c.) dose of formoterol (3 mg/kg body weight (bw) and pentoxifylline (10 mg/kg body weight (bw), dissolved in physiological solution) and the latter with the corresponding volume of solvent. On days two, four and seven after tumor inoculation, the animals were sacrificed.

In the treatment with different doses of formoterol the experimental groups were the following: tumor-bearing rats without treatment and tumor-bearing rats treated subcutaneously with different doses of formoterol (0.3, 1, 3, and 10 mg/kg body weight. On day seven after tumor inoculation, the animals were sacrificed.

In the treatment with low doses of formoterol the experimental groups were the following: control rats and tumor-bearing rats, two, four and seven days after tumor inoculation. Both groups were further divided into treated and untreated, the former being administered with a daily subcutaneously (s.c) dose of formoterol (0.3 mg/kg body weight (bw), dissolved in physiological solution) and the latter with the corresponding volume of solvent. On days two, four and seven after tumor inoculation, the animals were sacrificed.

In the treatment of muscular damage, the experimental groups were the following: control rats, rats whom a muscular damage had been performed with an acute injection of bupivacain (0.5%), rats whom a muscular damage had been performed with an acute injection of bupivacain (0.5%) and had been treated with formoterol the last seven days, rats whom a muscular damage had been performed with an acute injection of bupivacain (0.5%) and had been treated with formoterol the fourteen days. The formoterol treatment was with a daily subcutaneously dose of formoterol of 0.3 mg/kg body weight, dissolved in physiological solution.

In all the experiments, the tumor was harvested from the peritoneal cavity and its volume and cellularity evaluated. Blood was collected from the abdominal aorta into heparinized tubes and centrifuged (3500 g, 10 min, 4 °C) to obtain plasma. Tissues were rapidly excised, weighted, and frozen in liquid nitrogen.

Lewis lung carcinoma: This is a suitable model system to study the mechanisms involved in the establishment of cachexia. This tumor has been described as an anaplastic epidermoid with a marked hemorrhagic tendency, which regularly, spontaneously and consistently produces multiple lung metastasis. Its growth causes in the host rapid and progressive loss of body weight and tissue waste, particularly in skeletal muscle. Mice were divided into two groups, namely controls and tumor hosts. The tumor hosts received an inoculum of 5 x 10⁵ Lewis lung carcinoma cells obtained from exponential tumors, given intramuscularly (left thigh). The development of a nodule at the side of the injection, growing in size up to 20% of the animal weight, was considered as an index of effectiveness of the inoculation. Both groups were further divided into treated and untreated, the former being administered, during the last 9 days before sacrifice, a daily i.p. dose of formoterol (2 mg/kg bw, dissolved in physiological solution) and the latter a corresponding volume of solvent. On day 15 after tumor transplantation, the animals were weighted and anaesthetized with ketamine/xylazine mixture (i.p.) (Imalgene^{®} and Rompun^{®} respectively). The tumor was excised from the hind leg and its mass being determined. Samples of tissues were rapidly excised, weighted, and frozen in liquid nitrogen.

### RNA isolation and Northern blot analysis

Total RNA from EDL muscle was extracted using the acid guanidinium isothiocyanate/phenol/chloroform method. RNA samples (20 µg) were denatured, subjected to electrophoresis in 1.2% agarosa gels containing 6.3% formaldehyde and transferred to Hybond N membranes (Amersham). RNA was fixed to membrane by Genelinker. The RNA in gels and in filters was visualized with ethidium bromide and photographed by UV transillumination to ensure the integrity of RNA, to check the loading of equivalent amounts of RNA and to confirm proper transfer. RNA was transferred in 20x standard saline citrate (SSC; 0.15 M NaCI and 15 mM sodium citrate, pH 7.0). Hybridization was done at 65 °C overnight in the hybridization buffer (0.25 M Na₂HPO₄ / 7% SDS / 1 mM EDTA / 1 % BSA /10% dextran sulfate), and denatured labeled probes (10⁶-10⁷ cpm/ml) were added. Radiolabeled probes were prepared by the random primer method (Amersham). The ubiquitin probe used was a cDNA clone containing 12 pairs of the second ubiquitin coding sequence plus a complete third and fourth ubiquitin coding sequence and 120 base pairs of the 3'-untranslated region of the chicken polyubiquitin gene UBI. The membranes were also hybridized with cDNA probes encoding C8, C9, E2 conjugating enzyme, rat m-calpain and rat cathepsin B. Filters were exposed to Hyperfilm-MP (Amersham) at -80 °C for 1-4 days and the films quantified by scanning densitometry. Statistical analysis of the data was performed by means of the Student's t test.

### Determination of proteasome activity

The chymotrypsin-like activity of the muscle proteasome was determined by evaluating the cleavage of the specific fluorogenic substrate succinyl-Leu-Leu-Val-Tyr-7-amido-4-methylcoumarin (LLVY). The gastrocnemius muscle was homogenized in 20 mM TRIS-HCl pH 7.2 containing 0.1 mM EDTA, 1 mM 2-mercaptoethanol, 5 mM ATP, 20% glycerol, and 0.04% (v/v) Triton X-100. Muscle homogenates were then centrifuged (13000 g, 15 min, 4°C). The supernatant was collected and protein concentration was determined using the BCA assay (Pierce Chemical Co.). Aliquots of 40 µg protein were then incubated for 60 min at 37 °C in the presence of 40 µM LLVY. The incubation buffer for the evaluation of proteasome activity was 50 mM HEPES pH 8.0 containing 5 mM EGTA. Fluorescence was read with a spectrofluorometer (380 nm excitation and 460 nm emission; RF-5001 PC spectrofluorophotometer, Shimadzu). The activity, expressed as nkatal/mg protein referred to the total protein pool, was calculated by using free amidocoumarin as working standard. The final result is the difference between the nkatals obtained with and without the presence of 25 µM lactacystin, a specific proteasome inhibitor.

### Apoptosis assays

DNA fragmentation assay in tibialis muscle was performed as previously described (cf. Van Royen M. et al., "DNA fragmentation occurs in skeletal muscle during tumor growth: a link with cancer cachexia?" Biochem. Biophys. Res. Commun. 2000, vol. 270, pp. 533-7). Caspase-3 activity in tibialis muscle was determined using the BD Apoalert^{™} caspase-3 colorimetric assay kit (BD Biosciences Clontech, USA).

### Electrophoretic mobility shift analysis (EMSA)

Nuclear protein extracts from tibialis muscle were isolated as reported previously (cf. Blough E. et al., "Extraction of nuclear proteins from striated muscle tissue" Biotechniques 1999, vol. 26, pp. 202-4), and protein concentration was determined by BCA protein assay kit (Pierce, USA). Oligonucleotides corresponding to the consensus sequence to NF-κB (nuclear factor kappa B), AP-1 (activating protein-1) and C/EBP (CCAAT/enhancer binding protein) were end-labeled using [α³²P]dCTP and Klenow enzyme. The double stranded oligonucleotides end-labeled (30.000 cpm) were incubated for 10 min on ice with 150 µg of nuclear protein extract. Reactions were carried out in a final volume of 20 µl containing 12% glycerol, 12 mM Hepes (pH 7.9), 4 mM Tris-HCI (pH 7.9), 1 mM EDTA (pH 8), 1 mM dithiothreitol (DTT), 25 mM KCI, 5 mM MgCl₂, 40 µg/ml poly dl-dC (deoxyinosinic-deoxycytidylic acid) and protease inhibitors (PMSF, aprotinin and leupeptine). At the end of the incubation, mix samples were electrophoresed at 4 °C at 325 V for 60-80 min on a 7% nondenaturing polyacrylamide gel in 0.5x Tris-borate-EDTA. After electrophoresis, the gel was dried for 120 min in a BioRad Gel Dryer and exposed overnight time to an X-ray sensitive film (Hyperfilm-MP, Amersham Biosciences) at -80 °C with intensifying screens. The specificity of the NF-κB, AP-1 and C/EBP bands was confirmed by reactions with mutant oligonucleotides end-labeled.

### Determination of gene expression by real time PCR

Determination of gene expression of Pax-7 and myogenin were performed by real time PCR in tibialis muscle as previously reported (cf. Edstrom, E. et al., "Sarcopenia is not due to lack of regenerative drive in senescent skeletal muscle" Aging Cell 2005, vol. 4, pp. 65-77).

### Effect of formoterol treatment in rats bearing Yoshida AH-130 ascites hepatoma

Tumor cell number from hosts bearing Yoshida AH-130 model was not significantly affected by formoterol, as well as food intake (observed in tumor bearers). The hypophagia occurring during tumor growth persisted unchanged in treated animals (cf. TABLE 1).

Quite different was the pattern with regard to skeletal and heart muscles. Thus, as shown in TABLE 1, the implantation of Yoshida AH-130 ascites hepatoma resulted in a decrease in muscles weight (13% for gastrocnemius, 16% for tibialis, 11 % for soleus, 19% for EDL and 11 % for heart). Formoterol treatment resulted in an increase in most muscle types including gastrocnemius (28%), tibialis (25%), EDL (32%) and cardiac muscle (21 %) in the control group. In tumor-bearing animals, formoterol induced an increase in gastrocnemius (9%), tibialis (11 %), EDL (16%) and heart (11 %) weight thus preventing muscle wasting. Treatment with formoterol had therefore a clear protective effect, in terms of wet tissue weight, for the skeletal muscles and for the heart. Then, formoterol afforded a significant trophic action on muscles in non-tumor bearers as well, particularly on the gastrocnemius, tibialis, EDL and cardiac muscle, while the soleus was not significantly affected. Regarding adipose tissues, tumor growth caused large reductions in white adipose tissue mass (46%). A similar situation was observed for brown adipose tissue (53%). In the rat tumor model, formoterol treatment was associated with a tendency towards a decrease in white adipose tissue mass in both the control and tumor-bearing animals, although the results did not reach statistical significance, while it induced an increase in brown adipose tissue weight (cf. TABLE 1).

TABLE 1 shows food intake, tissue weights and tumor growth in rats bearing Yoshida AH-130 ascites hepatoma. Food intake is expressed in g and refers to the ingestion during the period of the experiment prior to sacrifice (7 days after tumor inoculation). Tissue weights are expressed as mg/100 g of initial body weight. Carcass (body without organs or tumor) is expressed as g/100 g of initial body weight. Tumor cell content is expressed in millions of cells. Results are mean ± SEM for the number of animals indicated in parentheses. C = control; F = formoterol-treated; TB = tumor-bearing. Statistical significance of the results (by Student's t test); C versus TB: *p<0.05, **p<0.01, ***p<0.001 Untreated versus F: †p<0.05; ††p<0.01, †††p<0.001.

**TABLE 1**

| | | C (4) | C+F (5) | TB (12) | TB+F (12) |
|---|---|---|---|---|---|
| food intake | | 121 ± 1 | 135 ± 2 †† | 105 ± 4 ** | 109 ± 5 ** |
| muscle weight: | | | | | |
| | - gastrocnemius | 673 ±12 | 859 ± 38 †† | 585 ± 10 *** | 639 ± 16 †† *** |
| | - tibialis | 219 ± 7 | 275 ± 12 †† | 184 ± 4 ** | 204 ± 4 †† *** |
| | - soleus | 46 ± 1 | 55 ± 4 | 41 ± 1 * | 40 ± 1 *** |
| | - EDL | 53 ± 2 | 70 ± 3 †† | 43 ± 1 *** | 50 ± 1 ††† *** |
| | - heart | 430 ± 17 | 520 ± 15 †† | 382 ± 7 ** | 426 ± 17 † ** |
| adipose tissues weight: | | | | | |
| | - white (dorsal) | 963 ± 103 | 759 ± 126 | 516 ± 90 * | 461 ± 49 * |
| | - brown (interscapular) | 260 ± 13 | 278 ± 21 | 123 ± 11 *** | 177 ± 11 † *** |
| carcass weight | | 92 ± 1 | 113 ± 11 | 80 ± 1 *** | 81 ± 1 *** |
| tumor cell content | | --- | --- | 3886 ± 444 (5) | 3403 ± 88 (5) |

The accelerated muscle protein breakdown in both the AH-130 and Lewis lung carcinoma hosts is basically achieved through activation of the ATP-ubiquitin-dependent proteolytic system. Thus, effects of formoterol on gene expression of different proteolytic systems present in skeletal muscle were investigated. Gene expression in gastrocnemius muscle of two polyubiquitin mRNA species (2.4 kb and 1.2 kb) and C8 proteasome subunit (both involved in the ATP-ubiquitin-dependent proteolytic pathway), m-calpain (calcium-dependent system) and cathepsin B (lysosomal system) were studied. As shown in TABLE 2, tumor-bearing animals showed an increased expression of the polyubiquitin genes in relation with the corresponding control animals: over 2-fold (2.4 kb) and 6-fold (1.2 kb) (cf. FIG. 1). When the tumor-bearing animals received formoterol, this activation was suppressed (TABLE 2). Interestingly, similar findings were found for the C8 and C9 proteasome subunit, and for the E2 conjugating enzyme, whose expression were significantly increased as a result of tumor growth and formoterol treatment suppressed this activation of gene expression (cf. TABLE 2 and FIG. 1). Concerning other proteolytic systems, tumor burden also resulted in an increase in the expression of the calcium-dependent system m-calpain (3-fold) but formoterol treatment did not abolish this activation in this tumor model tested (cf. TABLE 2 and FIG. 1). Finally, formoterol treatment decreased lysosomal cathepsin B expression in tumor-bearing rats (cf. TABLE 2).

TABLE 2 shows gene expression (mRNAs) of different proteolytic systems in gastrocnemius muscles from rats bearing Yoshida AH-130 ascites hepatoma. It was detected after hybridization with cDNA probes. Loading correction was carried out by performing blots with the rat 18S ribosomal subunit probe. Autoradiographs were subjected to scanning densitometry. Symbols are as in TABLE 1.

| TABLE 2 | | | | | |
|---|---|---|---|---|---|
| proteolytic system | | C | C+F | TB | TB+F |
| ubiquitin-dependent: | | | | | |
| | - ubiquitin 2.4 kb | 100 ± 12 (4) | 92 ± 12 (5) | 233 ± 23 (3) ** | 128 ± 19 (5) † |
| | - ubiquitin 1.2 kb | 52 ± 10 (3) | 57 ± 12 (5) | 311 ± 71 (3) * | 73 ± 15 (4) † |
| | - proteasome subunit C8 | 100 ± 9 (4) | 62 ± 7 (5) † | 155 ± 7 (5) ** | 78 ± 9 (5) ††† |
| | - proteasome subunit C9 | 100 ± 5 (4) | 104 ± 7 (5) | 126 ± 6 (5) * | 80 ± 4 (5) ††† |
| | - E2 conjugating enzyme 1,8 Kb | 100 ± 4 (4) | 91 ± 3 (5) | 163 ± 4 (5) *** | 136 ± 5 (5) †† |
| | - E2 conjugating enzyme 1,2 Kb | 100 ± 7 (4) | 88 ± 6 (5) | 157 ± 8 (5) *** | 128 ± 5 (5) † |
| | | | | | |
| calcium-dependent: | | | | | |
| | - m-calpain | 100 ± 13 (4) | 187 ± 14 (5) †† | 282 ± 33 (5) ** | 212 ± 9 (4) |
| lysosomal: | | | | | |
| | - cathepsin B | 100 ± 8 (4) | 106 ± 6 (5) | 113 ± 3 (5) | 65 ± 6 (5) ††† *** |

Taking into account the increase in proteasome activity previously described in skeletal muscle of tumor-bearing mice (cf. Whitehouse A.S. et al., "Mechanism of attenuation of skeletal muscle protein catabolism in cancer cachexia by eicosapentaenoic acid" Cancer Res. 2001, vol. 61, pp. 3604-9), it was decided to test if formoterol was also able to revert this activity in skeletal muscle of tumor-bearing rats. The most dominant catalytic activity (chymotrypsin-like activity) in gastrocnemius muscle of rats bearing the Yoshida AH-130 tumor was measured. The results obtained show a decrease of proteasome activity in both non tumor (64%) and tumor-bearing rats (47%) treated with formoterol (cf. TABLE 3).

TABLE 3 shows proteasome activity in skeletal muscle homogenates from tumor-bearing rats. The activities, expressed as nkatal/mg protein referred to the total protein pool, were calculated by using free amidocoumarin as working standard. The final result is the difference between the nkatals obtained with and without the presence of 25 µM lactacystin, a specific proteasome inhibitor. Symbols are as in TABLE 1.

**TABLE 3**

| Experimental group | Treatment | Proteasome activity |
|---|---|---|
| Control | None | 13.3 ± 2.5 (3) |
| | Formoterol | 4.8 ± 1.4 (3) * |
| Tumor | None | 20.3 ± 4.7 (3) |
| | Formoterol | 10.7 ± 2.4 (5) |

Another aspect that was evaluated was muscle apoptosis during cancer. The results presented in FIG. 2 show that tumor growth is associated with an increased rate of apoptosis both determined as DNA fragmentation (68%) (cf. FIG 2A and 2B) and as activation of caspase-3 (59%) (cf. FIG. 2C). Formoterol treatment completely abolished the increased rate of muscle apoptosis (cf. FIG. 2A, 2B and 2C).

The results shown in TABLE 4 show that none of the transcription factor studied (NF-κB, AP-1 and C/EBP) were involved in the development of muscle wasting during tumor growth.

TABLE 4 shows NF-κB, AP-1 and C/EBP DNA-binding activity in the tibialis muscles from rats bearing the Yoshida AH-130 ascites hepatoma. NF-κB, AP-1 and C/EBP DNA-binding were determined in nuclear extracts from tibialis muscle. Autoradiographs were subjected to scanning densitometry. Symbols are as in TABLE 1.

**TABLE 4**

| | C | C+F | TB | TB+F |
|---|---|---|---|---|
| Transcription factor | | | | |
| NF-κB | 100 ± 7 (4) | 118 ± 7 (5) | 100 ± 16 (4) | 93 ± 13 (5) |
| AP-1 | 100 ± 14 (4) | 85 ± 8 (5) | 112 ± 10 (5) | 105 ± 12 (4) |
| C/EBP | 100 ± 20 (4) | 128 ± 13 (4) | 122 ± 20 (4) | 107 ± 13 (5) |

Both UCP2 and UCP3 gene expression was increased in skeletal muscle as a consequence of tumor growth. Indeed, in gastrocnemius muscle UCP2 mRNA content was elevated 3-fold, while that of UCP3 was increased 5-fold. When the tumor-bearing rats received formoterol, this activation was diminished significantly in UCP2 expression and a great tendency was observed in UCP3 expression (cf. TABLE 5).

TABLE 5 shows gene expression (mRNA) of the uncoupling proteins (UCP2 and UCP3) in gastrocnemius muscles from rats bearing Yoshida AH-130 ascites hepatoma. It was detected after hybridization with cDNA probes. Loading correction was carried out by performing blots with the rat 18S ribosomal subunit probe. Autoradiographs were subjected to scanning densitometry. Symbols are as in TABLE 1.

**TABLE 5**

| | C | C+F | TB | TB+F |
|---|---|---|---|---|
| UCP2 | 100 ± 14 (3) | 106 ± 7 (5) | 317 ± 56 (4) * | 212 ± 31 (5)(p=0,1) |
| UCP3 | 100 ± 11 (4) | 84 ± 18 (4) | 573 ± 66 (4) *** | 352 ± 41 (4) † |

### Direct effect of formoterol in muscle incubations

The dissection and isolation of the EDL muscles was carried out under ketamine/xylazine mixture anesthesia. The isolated muscles were fixed to a stainless-steel clip in order to maintain the muscle under slight tension (making it comparable to resting length) during the incubation. Such muscles are able to maintain normal ATP and phosphocreatine concentrations during a 3 hours incubation period. The muscles were incubated in a shaking-thermostatized water bath at 35 °C for 3 hours in 2 ml of Krebs-Henseleit physiological saline pH 7.4, containing 5 mM glucose and 20 mM HEPES. After the addition of the muscles to the vials, the incubation started at a shaking rate of 45 cycles/min. Vials were gassed with O₂/CO₂ 19:1 during the whole incubation period. The muscles were preincubated for 60 minutes: 30 minutes in Krebs-Henseleit buffer and 30 minutes in supplemented medium containing either 10⁻⁴ M formoterol, 10⁻⁴ M clenbuterol or none, and then incubated for 120 minutes in fresh supplemented medium.

Total protein degradation by the isolated muscles was calculated as the rate of tyrosine released in the last two hours of incubation into the medium in the presence of 0.5 mM cycloheximide in order to block the reincorporation of tyrosine into tissue protein. Tyrosine was measured fluorimetrically. Statistical analysis of the data was performed by means of the Student's t test. As can be seen in TABLE 6, formoterol decreased the rate of protein degradation (measured as the release of tyrosine to the incubation medium) by 20%, thus confirming that the action of the molecule is based on a decrease in the proteolytic rate.

TABLE 6 shows proteolytic rates in isolated EDL muscles from control rats. They were measured in presence of cycloheximide (0.5 mM) and are expressed as nmols/g and 2 hours and are mean ± SEM for the number of animals indicated in parentheses. Statistical significance of the results (by Student's t test); β₂-agonist added versus none ***p<0.001.

**TABLE 6**

| Additions to incubation medium | Proteolytic rate |
|---|---|
| None | 225 ± 8.9 (7) |
| formoterol | 180 ± 5.6 (8) *** |
| clenbuterol | 176 ± 4.4 (8) *** |

In addition, the proteolytic rate was also significantly decreased (12%) in rats that had been previously pre-treated for six consecutive days with the β₂-agonist (cf. TABLE 7).

TABLE 7 shows proteolytic rates in isolated EDL muscles from rats given formoterol (2 mg/kg bw) treatment for six consecutive days prior to sacrifice. Rates were measured in presence of cycloheximide (0.5 mM) and are expressed as nmols/g and 2 hours. Statistical significance of the results (by Student's t test): treated versus control rats *p<0.05.

**TABLE 7**

| Treatment | Proteolytic rate |
|---|---|
| None | 182 ± 7 (12) |
| Treated-rats | 159 ± 5 (8) * |

In addition to the effects on the rate of protein degradation in incubated rat muscles, both formoterol and clenbuterol influenced the rate of protein synthesis which was increased by 20% both, for formoterol and clenbuterol (cf. TABLE 8). Total protein synthesis rate in isolated muscles was evaluated as previously described (cf. Garcia-Martinez C. et al., "Interleukin-6 does not activate protein breakdown in rat skeletal muscle" Cancer Lett. 1994, vol. 76, pp. 1-4). These results agree with similar observations made in previous studies involving β₂-agonists in incubated rat muscles (cf. Rehfeldt C. et al., "The effect of the beta-adrenergic agonist clenbuterol on the growth of skeletal muscles of rats" Arch. Tierernahr 1994, vol. 45, pp. 333-44).

TABLE 8 shows protein synthesis rate in isolated EDL muscles from control rats. Synthetic rates are expressed as nmols of [¹⁴C]phenylalanine incorporated per g and 30 min. Statistical significance of the results (by Student's t test): β₂-agonist added versus none *p<0.05.

**TABLE 8**

| Additions to incubation medium | Rate of protein synthesis |
|---|---|
| None | 7.82 ± 0.5 (6) |
| Formoterol | 9.36 ± 0.3 (7) * |
| Clenbuterol | 9.36 ± 0.5 (9) * |

It was demonstrated that the combination between formoterol (3 mg/kg bw) and pentoxifylline, inhibitor of the tumor necrosis factor-alpha synthesis (TNF-α) (cf. Combaret L. et al.,"Manipulation of the ubiquitin-proteasome pathway in cachexia: pentoxifylline suppresses the activation of 20S and 26S proteasomes in muscles from tumor-bearing rats" Mol Biol Rep 1999, vol. 26, pp.95-101) (10 mg/kg bw) resulted in a positive effect on the gain of muscular mass due to the cachexia (TABLE 9). This effect was observed the different days of tumor growth (days 2, 4 and 7 after tumor inoculation).

TABLE 9 (A, B and C) shows food intake, tissue weights and tumor growth in rats bearing Yoshida AH-130 ascites hepatoma. Food intake is expressed in g and refers to the ingestion during the period of the experiment prior to sacrifice (2, 4 and 7 days after tumor inoculation, TABLE 9A, 9B and 9C respectively). Tissue weights are expressed as mg/100 g of initial body weight. Carcass (body without organs or tumor) is expressed as g/100 g of initial body weight. Tumor cell content is expressed in millions of cells. Results are mean ± SEM for the number of animals indicated in parentheses. C = control; F = formoterol-treated; P= pentoxifylline-treated; TB = tumor-bearing. Statistical significance of the results (by Student's t test); C versus TB: *p<0.05, **p<0.01, ***p<0.001 Untreated versus F: tp<0.05; ttp<0.01, †††p<0.001.

**TABLE 9A (two days after tumor inoculation)**

| | | C (4) | C+F+P (5) | TB (5) | TB+F+P (5) |
|---|---|---|---|---|---|
| Food intake | | 35 ± 1 | 29 ± 1 ††† | 32 ± 1 | 26 ± 1 † |
| muscle weights: | | | | | |
| | - gastrocnemius | 484 ±13 | 533 ± 11 † | 503 ± 9 | 553 ± 17 † |
| | - tibialis | 167 ± 6 | 185 ± 3 † | 171 ± 4 | 183 ± 3 † |
| | - soleus | 39 ± 2 | 44 ± 2 | 40 ± 1 | 43 ± 1 |
| | - EDL | 39 ± 1 | 43 ± 1 † | 40 ± 1 | 43 ± 1 † |
| | - heart | 410 ± 17 | 430 ± 20 | 351 ± 8 ** | 424 ± 10 ††† |
| carcass weight | | 93 ± 1 | 93 ± 1 | 93 ± 1 | 92 ± 1 |

**TABLE 9B (four days after tumor inoculation)**

| | | C (3) | C+F+P (5) | TB (7) | TB+F+P (5) |
|---|---|---|---|---|---|
| Food intake | | 52 ± 1 | 60 ± 3 † | 61 ± 3 | 65 ± 3 |
| muscle weights: | | | | | |
| | - gastrocnemius | 590 ±16 | 597 ± 14 | 517 ± 9 *** | 594 ± 11 ††† |
| | - tibialis | 195 ± 3 | 198 ± 6 | 173 ± 4 ** | 203 ± 3 ††† |
| | - soleus | 43 ± 1 | 45 ± 2 | 41 ± 1 | 42 ± 1 |
| | - EDL | 46 ± 1 | 46 ± 1 | 40 ± 1 | 46 ± 1 |
| | - heart | 402 ± 9 | 517 ± 22 †† | 387 ± 17 | 430 ± 9 (p=0,08) ** |
| carcass weight | | 101 ± 2 | 97 ± 2 | 96 ± 2 | 97 ± 1 |
| tumour cell content | | --- | --- | 1886 ± 197 | 1059 ± 435 |

**TABLE 9C (seven days after tumour inoculation)**

| | | C (4) | C+F+P (5) | TB (5) | TB+F+P (6) |
|---|---|---|---|---|---|
| Food intake | | 123 ± 1 | 148 ± 1 ††† | 98 ± 3 *** | 84 ± 1 ††† *** |
| muscle weights: | | | | | |
| | - gastrocnemius | 651 ±10 | 745 ± 15 †† | 482 ± 22 *** | 596 ± 20 *** |
| | - tibialis | 235 ± 6 | 247 ± 3 | 161 ± 5 *** | 188 ± 8 † *** |
| | - soleus | 51 ± 2 | 52 ± 2 | 38 ± 1 ** | 41 ± 2 ** |
| | -EDL | 53 ± 2 | 60 ± 2 † | 38 ± 1 *** | 44 ± 1 † *** |
| | - heart | 430 ± 29 | 484 ± 23 | 330 ± 27 * | 415 ± 8 * |
| carcass weight | | 107 ± 8 | 99 ± 4 | 89 ± 6 | 95 ± 1 |
| tumour cell content | | --- | --- | 3596 ± 531 | 3868 ± 810 |

The treatment with different doses of formoterol (0.3, 1, 3 y 10 mg/kg bw) were similar, then at very low doses formoterol is effective against cachexia (TABLE 10).

TABLE 10 shows food intake, tissue weights and tumor growth in rats bearing Yoshida AH-130 ascites hepatoma. Food intake is expressed in g and refers to the average of daily ingestion. All the other parameters are expressed as in TABLE 9. F = formoterol-treated; TB = tumor-bearing. Statistical significance of the results (by Student's t test); Untreated versus F: tp<0.05; ††p<0.01, †††p<0.001.

**TABLE 10**

| | | TB (6) | TB + F 0.3 (5) | TB + F 1 (4) | TB + F 3 (5) | TB + F10 (5) |
|---|---|---|---|---|---|---|
| Food intake | | 14 ± 1 | 11 ± 1 | 8 ± 1 ††† | 9 ± 1 ††† | 10 ± 1 ††† |
| muscle weights: | | | | | | |
| | - gastrocnemius | 486 ± 7 | 563 ± 23 †† | 518 ± 34 | 557 ± 15 ††† | 537 ± 5 ††† |
| | -tibialis | 154 ± 3 | 172 ± 7 † | 166 ± 6 | 161 ± 5 | 159 ± 5 |
| | - soleus | 35 ± 1 | 37 ± 1 | 33 ± 2 | 37 ± 2 | 37 ± 2 |
| | -EDL | 36 ± 1 | 41 ± 1 †† | 39 ± 2 | 38 ± 1 | 40 ± 1 † |
| | - heart | 278 ± 6 | 322 ± 9 †† | 320 ± 13 †† | 341 ± 12 ††† | 351 ± 11 ††† |
| carcass weight | | 78 ± 1 | 81 ± 2 | 77 ± 2 | 80 ± 1 | 77 ± 1 |
| tumour cell content | | 3842 ± 251 | 3294 ± 272 | 3644 ± 356 | 3787 ± 424 | 3675 ±146 |

According to the last results, it was decided to study the effect of formoterol at low doses (0.3 mg/kg bw) on cancer cachexia at different days of tumor growth (days 2, 4 and 7 after tumor inoculation) and it was confirmed that this β₂-adrenoceptor-selective agonist is also effective at low doses (TABLE 11 A, B and C).

TABLE 11 (A, B and C) shows food intake, tissue weights and tumor growth in rats bearing Yoshida AH-130 ascites hepatoma. All the other parameters are expressed as in TABLE 9.

**TABLE 11 A (two days after tumour inoculation)**

| | | C (5) | C+F (5) | TB (7) | TB+F (7) |
|---|---|---|---|---|---|
| Food intake | | 38 ± 1 | 34 ± 1 ††† | 36 ± 1 * | 35 ± 1 |
| muscle weights: | | | | | |
| | - gastrocnemius | 540 ±12 | 563 ± 18 | 523 ± 9 | 539 ± 8 † |
| | - tibialis | 177 ± 3 | 186 ± 5 | 179 ± 5 | 182 ± 4 |
| | - soleus | 41 ± 2 | 45 ± 2 | 39 ± 2 | 40 ± 1 * |
| | -EDL | 45 ± 3 | 50 ± 2 | 44 ± 1 | 49 ± 1 † |
| | - heart | 366 ± 11 | 426 ± 6 †† | 377 ± 11 | 403 ± 10 (p=0.1) |
| carcass weight | | 90 ± 1. | 92 ± 1 | 89 ± 1 | 88 ± 1 |

**TABLE 11 B (four days after tumour inoculation)**

| | | C (5) | C+F (5) | TB (7) | TB+F (7) |
|---|---|---|---|---|---|
| Food intake | | 75 ± 1 | 71 ± 1 †† | 69 ± 2* | 65 ± 1 ** |
| muscle weigths: | | | | | |
| | - gastrocnemius | 583 ±9 | 629 ± 9 †† | 553 ± 6 * | 598 ± 16 †† |
| | - tibialis | 191 ± 3 | 210 ± 5 † | 181 ± 4 | 199 ± 4 † |
| | - soleus | 44 ± 1 | 43 ± 2 | 41 ± 1 | 43 ± 1 |
| | - EDL | 48 ± 1 | 52 ± 1 † | 46 ± 1 | 51 ± 2 † |
| | - heart | 396 ± 7 | 431 ± 5 †† | 369 ± 6 * | 417 ± 18 † |
| carcass weigth | | 102 ± 1 | 106 ± 1 †† | 94 ± 1 *** | 97 ± 1 *** |
| tumour cell content | | --- | --- | 1346 ± 224 | 1065 ± 95 |

**TABLE 11C (seven days after tumour inoculation)**

| | | C (5) | C+F (5) | TB (6) | TB+F (7) |
|---|---|---|---|---|---|
| Food intake | | 133 ± 1 | 131 ± 2 | 106 ± 1 *** | 108 ± 1 *** |
| muscle weights: | | | | | |
| | - gastrocnemius | 718 ±10 | 759 ± 16 † | 505 ± 17 *** | 611 ± 18 †† *** |
| | - tibialis | 236 ± 6 | 266 ± 5 ††† | 171 ± 5 *** | 200 ± 9 † *** |
| | - soleus | 52 ± 2 | 52 ± 2 | 41 ± 3 ** | 44 ± 1 ** |
| | - EDL | 59 ± 2 | 60 ± 2 | 42 ± 2 *** | 51 ± 2 † * |
| | - heart | 449 ± 6 | 504 ± 10 †† | 372 ± 19 ** | 403 ± 9 *** |
| carcass weight | | 118 ± 1 | 124 ± 2 † | 88 ± 3 *** | 94 ± 3 (p=0.1) *** |
| tumour cell content | | --- | ---- | 3386 ± 163 | 2871 ± 271 |

Muscular regeneration is a process that implies satellite cells activation, muscle precursors cells that lie adjacent to the skeletal muscle fibers. The muscle was damaged by injecting bupivacain intramuscularly (0.5%). This compound, after muscle damage, stimulates muscular regeneration. Moreover, the effect of two treatments with formoterol was checked (0.3 mg/kg bw): 7 or 14 days of treatment. TABLE 12 shows the anabolic effect of the formoterol treatments on the tibialis muscle previously damaged with bupivacain.

TABLE 12 shows the weight of tibialis muscles previously injected with bupivacain and later treated with formoterol during 7 or 14 days. Muscle weights are expressed as mg/100 g of initial body weight. Results are mean ± SEM for the number of animals indicated in parentheses. C = control; F = formoterol-treated; B= bupivacain-treated; d7 = 7 days of treatment; d14 = 14 days of treatment. Statistical significance of the results (by Student's t test); C versus treated: *p<0.05, **p<0.01, bupivacain versus formoterol: †<0.05.

**TABLE 12**

| | C(4) | C+ B (4) | C+B+Fd7(3) | C + B+ F d14 (4) |
|---|---|---|---|---|
| - tibialis | 249 ± 4 | 265 ± 4 * | 297 ± 13 * † | 304 ± 10 † |

Formoterol treatment induced a higher recovery of the muscle weight compared with the recovery due to the damage inductor and later stimulation of muscle regeneration (bupivacain). For this reason, gene expression of some muscle regeneration markers was studied: genes expressed during satellite cell activation, such as Pax-7 (early activation marker) and myogenin (final activation marker). The results are detailed in TABLE 13.

TABLE 13 shows gene expression of two satellite cell activation markers: PAx-7 and myogenin. Results are mean ± SEM for the number of animals indicated in parentheses. C = control; F = formoterol-treated; B= bupivacain-treated; d7 = 7 days of treatment; d14 = 14 days of treatment. Statistical significance of the results (by Student's t test); C versus treated: *p<0.05, **p<0.01. Bupivacain versus formoterol: t<0.05.

**TABLE 13**

| | C (3) | C+ B (3) | | C + B+ F d7 (3) | C + B+ F d14 (3) |
|---|---|---|---|---|---|
| - Pax-7 | 100 ± 11 | 105 ± 11 | | 147 ± 10 | 203 ± 4 |
| | | † | | | †† |
| - Myogenin | 100 ± 36 | 142 ± 77 | 131 ± 49 | | 190 ± 58 |

### Effect of formoterol treatment in mice bearing Lewis lung carcinoma

Formoterol treatment did not alter either the weight of the solid tumor from hosts bearing Lewis lung carcinoma model, as well as the food (controls and tumor bearers). The hypophagia occurring during tumor growth persisted unchanged in treated animals (TABLE 14).

In Lewis lung carcinoma model tumor burden induced a great decrease in muscle weights (13% for gastrocnemius, 21 % for tibialis and 18% for soleus) (TABLE 14). Formoterol treatment increased the muscle weights of gastrocnemius (19%), tibialis (13%) and soleus (28%) significantly in the non tumor-bearing animals. Interestingly, treatment with the formoterol had a significant protective effect on skeletal muscle weights -gastrocnemius (11 %), tibialis (11%) and soleus (56%)- and in cardiac muscle (24%) in tumor-bearing animals. Regarding adipose tissues, tumor growth caused large reductions in white adipose tissue mass in the Lewis lung carcinoma model (76%). A similar situation was observed for brown adipose tissue (39%). In the Lewis lung carcinoma model, formoterol did not alter the white adipose tissue mass in either control or tumor-bearing animals, but it induced an increase in brown adipose tissue mass in both control and tumor-bearers (TABLE 14).

TABLE 14 shows food intake, tissue weights and tumor growth in mice bearing Lewis lung carcinoma. Food intake is expressed in g and refers to the ingestion during the period of the experiment prior to sacrifice which place 15 days after tumor inoculation. Tissue weights are expressed as mg/100 g of initial body weight. Carcass (body without organs or tumor) is expressed as g/100 g of initial body weight. Tumor weight is expressed in g. Symbols are as in TABLE 1.

**TABLE 14**

| | | C (7) | C+F (6) | TB (8) | TB+F (7) |
|---|---|---|---|---|---|
| food intake | | 46 ± 1 | 53 ± 4 | 51 ± 3 | 53 ± 2 |
| muscle weight: | | | | | |
| | - gastrocnemius | 563 ± 4 | 669 ± 13 ††† | 492 ± 9 *** | 548 ± 13 †† *** |
| | - tibialis | 192 ± 2 | 217 ± 4 ††† | 152 ± 3 *** | 169 ± 3 †† *** |
| | - soleus | 28 ± 1 | 36 ± 1 ††† | 23 ± 2 * | 36 ± 2 ††† |
| | - EDL | 40 ± 1 | 42 ± 2 | 35 ± 2 | 40 ± 2 |
| | - heart | 432 ± 15 | 462 ± 31 | 401 ± 17 | 496 ± 40 † |
| adipose tissues weight: | | | | | |
| | - white (dorsal) | 238 ±18 | 217 ± 25 | 58 ± 12 *** | 68 ± 9 *** |
| | - brown (interscapular) | 208 ±12 | 249 ± 6 † | 127 ± 5 *** | 153 ± 13 † *** |
| carcass weight | | 80 ± 1 | 87 ± 1 ††† | 69 ± 1 *** | 73 ± 2 *** |
| tumor weight | | --- | --- | 4.26 ± 0.3 | 4.58 ± 0.3 |

As shown in TABLE 15, tumor-bearing mice showed an increased expression of the polyubiquitin genes in relation with the corresponding control animals: over 2-fold (2.4 kb) and 3-fold (1.2 kb) (FIG. 3). When the tumor-bearing mice received formoterol, this activation was suppressed (TABLE 15). Interestingly, similar findings were found for the C8 and C9 proteasome subunit, whose expression was significantly increased as a result of tumor growth, and formoterol treatment suppressed this activation of gene expression (TABLE 15 and FIG. 3). Concerning other proteolytic systems, tumor burden also resulted in an increase in the expression of the calcium-dependent system m-calpain (2-fold), but formoterol treatment did not abolish this activation in Lewis lung carcinoma model (TABLE 15 and FIG. 3). Finally, the slight increase in mRNA content observed in the mice bearing Lewis lung carcinoma in the lysosomal cathepsin B was not reverted by formoterol treatment (TABLE 15 and FIG. 3).

TABLE 15 shows gene expression (mRNAs) of the different proteolytic systems in gastrocnemius muscles from mice bearing Lewis lung carcinoma. It was detected after hybridization with cDNA probes. Loading correction was carried out by performing blots with the rat 18S ribosomal subunit probe. Autoradiographs were subjected to scanning densitometry. Symbols are as in TABLE 1.

**TABLE 15**

| proteolytic system | | C | C+F | TB | TB+F |
|---|---|---|---|---|---|
| ubiquitin-dependent: | | | | | |
| | - ubiquitin 2.4 kb | 100 ± 9 (5) | 50 ± 9 (5) †† | 227 ± 59 (4) * | 90 ± 21 (6) †† |
| | - ubiquitin 1.2 kb | 36 ± 3 (5) | 25 ± 5 (5) | 113 ± 14 (4) *** | 27 ± 5 (6) ††† |
| | - proteasome subunit C8 | 100 ± 8 (5) | 116 ± (5) | 9 177 ± 11 (5) *** | 129 ± 12 (6)† |
| | - proteasome subunit C9 | 100 ± 10 (5) | 114 ± 7 (4) | 145 ± 3 (5) ** | 101 ± 12 (5) †† |
| | | | | | |
| calcium-dependent: | | | | | |
| | - m-calpain | 100 ± 21 (5) | 136 ± 20 (5) | 222 ± 27 (5) *** | 254 ± 33 (7) * |
| lysosomal: | | | | | |
| | - cathepsin B | 100 ± 14 (5) | 137 ± 6 (3) | 157 ± 11 (5) * | 161 ± 1 (7) |

Both UCP2 and UCP3 gene expression was increased in gastrocnemius muscle as a consequence of Lewis lung tumor growth. In gastrocnemius muscle UCP2 mRNA content was elevated 3-fold, while that of UCP3 was increased 2-fold (without reaching significance). When the turnor-bearing mice received formoterol, this activation was abolished in both genes expression (TABLE 16).

TABLE 16 shows gene expression (mRNA) of the uncoupling proteins (UCP2 and UCP3) in gastrocnemius muscles from mice bearing Lewis lung carcinoma. It was detected after hybridization with cDNA probes. Loading correction was carried out by performing blots with the rat 18S ribosomal subunit probe. Autoradiographs were subjected to scanning densitometry. Symbols are as in TABLE 1.

**TABLE 16**

| | C | C+F | TB | TB+F |
|---|---|---|---|---|
| UCP2 | 100 ± 25 (4) | 102 ± 17 (6) | 310 ± 37 (5) ** | 175 ± 21 (7) † |
| UCP3 | 100 ± 11 (3) | 94 ± 10 (5) | 174 ± 28 (4) (p=0,08) | 102 ± 13 (6) † |

## Claims

1. Use of formoterol or a pharmaceutically acceptable salt, or a pharmaceutically acceptable solvate of either for the preparation of a medicament for the prophylactic and/or therapeutic treatment of muscle wasting and/or a cachectic syndrome, either or both being associated with a catabolic state, in a mammal

2. Use according to claim 1, wherein the catabolic state is Crohn's disease, cancer or AIDS.

3. Use according to claim 1, wherein the catabolic state is sepsis, diabetes or an immobilization state.

4. Use according to claim 1, wherein the catabolic state is severe bum or due to weightlessness or ageing.

5. Use according to claim 1, wherein the catabolic state is chronic obstructive pulmonary disease, a cardiovascular pathology or a hepatic pathology.

6. Use according to claim 1, wherein the catabolic state is rheumatoid arthritis, a nutrition disorder or acidosis.

7. Use according to claim 1, wherein the catabolic state is a neuromuscular disease, Alzheimer's disease or Cushing's syndrome.

8. Use according to claim 1, wherein the catabolic state is hyperthyroidism, hyperadrenocortisolism or a muscle atrophy and/or distrophy.

9. Use according to claim 1, wherein the catabolic state is ulcerative colitis, chronic fatigue syndrome, renal failure or liver cirrhosis.

10. Use according to any preceding claim, wherein the use is of formoterol fumarate or formoterol fumarate dihydrate.

11. Use according to any preceding claim, wherein the mammal is a human.

12. Use according to any preceding claim, wherein the medicament is to be administered by the oral route.

13. Use according to any of claims 1 to 11, wherein the medicament is to be administered by the nasal route.

14. Use according to any of claims 1 to 11, wherein the medicament is to be administered by the parenteral route.

15. Use according to any of claims 1 to 11, wherein the medicament is to be administered by the subcutaneous route.

16. Use according to any of claims 1 to 11, wherein the medicament is to be administered by the topical route.

## Patentansprüche

1. Verwendung von Formoterol oder einem pharmazeutisch verträglichen Salz oder einem pharmazeutisch verträglichen Solvat davon für die Herstellung eines Medikamentes für die prophylaktische und/oder therapeutische Behandlung von Muskelschwund und/oder einem Kachexie-Syndrom, entweder eines oder beide einen katabolischen Zustand bei einem Säugetier zugeordnet.

2. Verwendung gemäss Anspruch 1, bei dem der katabolische Zustand Morbus Crohn, Krebs oder Aids ist.

3. Verwendung gemäss Anspruch 1, bei dem der katabolische Zustand Sepsis, Diabetes oder ein Immobilisierungszustand ist.

4. Verwendung gemäss Anspruch 1, bei dem der katabolische Zustand eine schwere Verbrennung oder Schwerelosigkeit oder Alterung ist.

5. Verwendung gemäss Anspruch 1, bei dem der katabolische Zustand die chronische obstruktive Lungenerkrankung (COPD), eine kardiovaskuläre Pathologie oder eine hepatitische Pathologie ist.

6. Verwendung gemäss Anspruch 1, bei dem der katabolische Zustand rheumatoide Arthritis, eine Ernährungsstörung oder Azidose ist.

7. Verwendung gemäss Anspruch 1, bei dem der katabolische Zustand eine neuromuskuläre Krankheit, die Alzheimer-Krankheit oder das Cushing-Syndrom ist.

8. Verwendung gemäss Anspruch 1, bei dem der katabolische Zustand Hyperthyroidismus, Hyperadrenocortisolismus oder eine Muskelatrophie und/oder -dystrophie ist.

9. Verwendung gemäss Anspruch 1, bei dem der katabolische Zustand eine Geschwür-Kolitis, chronisches Ermüdungssyndrom, Nierenversagen oder eine Leberzirrhose ist.

10. Verwendung gemäss einem der vorstehenden Ansprüche, bei dem die Verwendung durch Formoterol-Fumarat oder Formoterol-Fumarat-Dihydrat geschieht.

11. Verwendung gemäss einem der vorstehenden Ansprüche, bei dem das Säugetier ein Mensch ist.

12. Verwendung gemäss einem der vorstehenden Ansprüche, bei dem das Medikament über den oralen Weg verabreichbar ist.

13. Verwendung gemäss einem der Ansprüche 1 bis 11, bei dem das Medikament über den nasalen Weg verabreichbar ist.

14. Verwendung gemäss einem der Ansprüche 1 bis 11, dem das Medikament durch den parenteralen Weg verabreichbar ist.

15. Verwendung gemäss einem der Ansprüche 1 bis 11, bei dem das Medikament über den subkutanen Weg verabreichbar ist.

16. Verwendung gemäss einem der Ansprüche 1 bis 11, bei dem das Medikament über den topischen Weg verabreichbar ist.

## Revendications

1. Utilisation de formotérol ou d'un sel pharmaceutiquement acceptable de celui-ci ou d'un solvate pharmaceutiquement acceptable de celui-ci pour la préparation d'un médicament pour le traitement prophylactique et/ou thérapeutique de l'atrophie musculaire et/ou d'un syndrome de cachexie, soit l'un ou les deux associé à un état catabolique chez un mammifère.

2. Utilisation selon la revendication 1, où l'état catabolique est la maladie de Crohn, le cancer ou le SIDA.

3. Utilisation selon la revendication 1, où l'état catabolique est la septicémie, le diabète ou un état d'immobilisation.

4. Utilisation selon la revendication 1, où l'état catabolique est une brulure importante ou du à l'apesanteur ou au vieillissement.

5. Utilisation selon la revendication 1, où l'état catabolique est la broncho-pneumopathie chronique obstructive (BPCO), une pathologie cardiovasculaire ou une pathologie hépatique.

6. Utilisation selon la revendication 1, où l'état catabolique est une polyarthrite rhumatoïde, une perturbation de l'alimentation ou une acidose.

7. Utilisation selon la revendication 1, où l'état catabolique est une maladie neuromusculaire, la maladie d'Alzheimer ou le syndrome de Cushing.

8. Utilisation selon la revendication 1, où l'état catabolique est le hyperthyroïdisme, le hyperadrenocortisolisme ou une atrophie musculaire et/ou une dystrophie musculaire.

9. Utilisation selon la revendication 1, où l'état catabolique est une rectocolite hémorragique, un syndrome de fatigue chronique, une insuffisance rénale aiguë ou une cirrhose du foie.

10. Utilisation selon l'une des revendications précédentes, où l'utilisation s'effectue par du formotérol fumarate ou du formotérol fumarate dihydrate.

11. Utilisation selon l'une des revendications précédentes, où le mammifère est un humain.

12. Utilisation selon l'une des revendications précédentes, où le médicament est à administrer par voie orale.

13. Utilisation selon l'une des revendications 1 à 11, où le médicament est à administrer par voie nasale.

14. Utilisation selon l'une des revendications 1 à 11, où le médicament est à administrer par voie parentérale.

15. Utilisation selon l'une des revendications 1 à 11, où le médicament est à administrer par voie subcutanée.

16. Utilisation selon l'une des revendications 1 à 11, où le médicament est à administrer par voie topique.
